Europäisches Patentamt

European Patent Office

Office européen des brevets

(19)

(11) EP 1 505 161 A2

(12) EUROPEAN PATENT APPLICATION

(43) Date of publication:
09.02.2005 Bulletin 2005/06

(51) Int Cl.$^7$: C12Q 1/70

(21) Application number: 04253110.3

(22) Date of filing: 27.05.2004

(84) Designated Contracting States:
AT BE BG CH CY CZ DE DK EE ES FI FR GB GR
HU IE IT LI LU MC NL PL PT RO SE SI SK TR
Designated Extension States:
AL HR LT LV MK

(30) Priority: 08.08.2003 US 455041

(71) Applicant: Yeung, Wah Hin Alex
Hong Kong (CN)

(72) Inventor: Yeung, Wah Hin Alex
Hong Kong (CN)

(74) Representative: Evans, Claire
Marks & Clerk
Incorporating Edward Evans Barker
Clifford's Inn
Fetter Lane
London EC4A 1BZ (GB)

(54) **Detection of polymorphisms in Epstein-Barr virus (EBV) DNA for the prediction and detection of EBV-associated cancers**

(57) The present invention features methods for diagnosing, detecting, monitoring and determining the prognosis of Epstein Barr virus associated cancers in a patient by detecting or measuring EBV DNA present in the serum or plasma of the patient followed by EBV subtyping of polymorphisms. The sensitivity of the circulating EBV DNA serves as a good screening tool while the EBV subtyping of polymorphism confirms the prognosis or diagnosis of EBV associated malignancies. The present invention also features diagnostic kits comprising suitable reagents for the above tests.

**Fig. 1**. *Diagram of Zp ( in $^\varepsilon$ ), the promoter of the Epstein-Barr virus BZLF1 gene. The different polymorphisms as described by Gutierrez et al indicated here by Zp-V3 (in * ) and Zp-V4 ( in $^\#$ ), Zp-P and Zp-V3 (malignant subtypes) and Zp-V4 (benign subtype).*

EP 1 505 161 A2

SEQUENCE LISTING

<110> Yeung, Wah Hin Alex
       Lo, Yuk Ming Dennis

<120> Combination of circulating epstein-barr virus (EBV) DNA in the serum or
plasma of patients and a method to assess EBV subtypes for the prediction and
detection of epstein-barr virus associated cancers

<130> 01/MED/070-Continuation In Part

<140> US 10/455,041
<141> 2003-06-03

<150> US 10/057,579
<151> 2002-01-25

<150> US 60/265,568
<151> 2001-01-31

<160> 6

<170> PatentIn version 3.1

<210> 1
<211> 19
<212> DNA
<213> Artificial

<220>
<223> BamHI-W system amplification primer W-44F

<400> 1
cccaacactc caccacacc                                                    19

<210> 2
<211> 20
<212> DNA
<213> Artificial

<220>
<223> BamHI-W system amplification primer W-119R

<400> 2
tcttaggagc tgtccgaggg                                                   20

<210> 3
<211> 27
<212> DNA
<213> Artificial

<220>
<223> BamHI-W system dual - labeled fluorescent probe W-67T

```
<220>
<221>  misc_feature
<222>  (1)..(1)
<223>  n = FAM - labeled c


<220>
<221>  misc_feature
<222>  (27)..(27)
<223>  n = TAMRA - labeled c


<400>  3
nacacactac acacacccac ccgtctn                                        27


<210>  4
<211>  20
<212>  DNA
<213>  Artificial

<220>
<223>  EBNA-1 system amplification primer EBNA-1162F

<400>  4
tcatcatcat ccgggtctcc                                                20


<210>  5
<211>  21
<212>  DNA
<213>  Artificial

<220>
<223>  EBNA-1 system amplification primer EBNA-1229R

<400>  5
cctacagggt ggaaaaatgg c                                              21


<210>  6
<211>  22
<212>  DNA
<213>  Artificial

<220>
<223>  EBNA-1 system dual-labeled fluorescent probe EBNA-1186T

<220>
<221>  misc_feature
<222>  (1)..(1)
<223>  n = FAM - labeled c


<220>
<221>  misc_feature
```

```
<222>  (22)..(22)
<223>  n = TAMRA - labeled a


<400>  6
ngcaggcccc ctccaggtag an                                    22
```

**Description**

**FIELD OF THE INVENTION**

This invention relates to the discovery that Epstein Barr virus may be found in the cell free fluid of a patient's blood and when such virus is found, the patients may be suffering from Epstein Barr virus associated cancer. A new method to detect EBV subtypes that are more cancer prone will enable the prediction and diagnosis of such cancers.

BACKGROUND OF THE INVENTION

It is known that tumour-derived DNA can be released by cancer cells of a variety of tumours (Anker *et al.*, Cancer Metastasis Rev. 18: 65-73 (1999)). Examples include oncogene mutations from pancreatic carcinoma (Anker *et al.*, Gastroenterology. 112: 4-1120 (1997)), microsatellite alterations in lung cancer (Chen *et al.*, Nature Medicine. 2: 3-1035 (1996)) and epigenetic changes from liver cancer (Wong *et al.*, Cancer Res. 59: 3 (1999)). In addition, virus DNA has been found in the circulation of a number of cancers known to be associated with virus infection. Examples include Epstein-Barr virus (EBV) DNA from nasopharyngeal cancer (Mutirangura *et al.*, Clin Cancer Res. 4: 665-9 (1998); Lo *et al.*, Cancer Res. 59: 1188-91 (1999)) and certain lymphomas (Lei *et al.*, Br J Haematol. 111: 239-46 (2000); Gallagher *et al.*, Int J Cancer. 84: 442-8 (1999); Drouet *et al.*, J Med Virol. 57: 383-9 (1999)), and human papillomavirus DNA from head and neck cancer (Capone *et al.*, Clin Cancer Res. 6: 4171-5 (2000)).

Recently, much interest has been focused on the presence of tumor-derived DNA in the plasma and serum of cancer patients (Chen, X.Q. *et al.*, Nat. Med., 2: 1033-1035 (1996); Nawroz, H. *et al.*, Nat. Med., 2: 1035-1037 (1996)). For virally-associated cancers, cell-free tumor-associated viral DNA has been detected in the plasma and serum of patients (Mutirangura, A. *et al.*, Cancer Res., 4: 665-669 (1998); Lo, Y.M.D. *et al.*, Clin. Cancer Res. 59: 1188-1191 (1999); Capone, R.B. Clin. Cancer Res., 6: 4171-4175 (2000)). One important virus which has been associated with many types of malignancy is the Epstein-Barr virus (EBV) (Cohen, J.I.N. Engl. J. Med., 343: 481-492 (2000)). Epstein-Barr virus (EBV) is a human herpesvirus that infects the majority of the human population. EBV is commonly transmitted by saliva and established latent infection in B lymphocytes where it persists for the lifetime of the host. In this regard, circulating EBV DNA has been detected in the plasma and serum of patients with nasopharyngeal carcinoma (NPC) (Mutirangura, A. *et al.*, Cancer Res., 4: 665-669 (1998); Lo, Y.M.D. *et al.*, Clin. Cancer Res., 59: 1188-1191 (1999)) and certain lymphoid malignancies (Lei, K.I. *et al.*, Br. J. Haematol., 111: 239-246 (2000); Drouet, E. *et al.*, J. Med. Virol., 57: 383-389 (1999); Gallagher, A. *et al.*, Int. J. Cancer, 84: 442-448 (1999)).

EBV infection has also been reported to be associated with a proportion of gastric carcinomas (Shibata, D. *et al.*, Am. J. Pathol., 140: 769-774 (1992)). In Hong Kong, approximately 10% of gastric carcinoma cases have been found to be associated with EBV infection (Yuen, S.T. *et al.*, Am. J. Surg. Pathol., 18: 1158-1163 (1994)).

The present invention provides methods for detecting EBV DNA in the sera of patients. If EBV DNA were found by this method and no cancer is found clinically, another step is taken whereby the different subtypes of EBV with single nucleotide polymorphism are confirmed. Patients with the benign subtype will be unlikely to develop into EBV associated cancer. Those with the more cancerous subtypes of EBV, however, will be more prone to develop such cancer later.

**BRIEF SUMMARY OF THE INVENTION**

In a first aspect, the present invention features methods for diagnosing, detecting, monitoring and determining the prognosis of EBV associated cancers apart from head, neck and lymphoid malignancies in a patient. The methods feature detecting or determining the amount of Epstein Barr Virus DNA (EBV DNA) present in the serum or plasma of such patients. Accordingly, the present invention have broad applicability in clinical medicine especially latent EBV infection occur over 90% of some populations.

The methods according to the present invention are also applicable for diagnosing, detecting, monitoring and determining the prognosis of any EBV associated cancers including lymphomas.

The methods according to the present invention generally comprise the steps of (1) obtaining a blood sample from a patient, (2) extracting DNA from the blood sample, (3) measuring the amount of circulating EBV DNA present in the blood sample, and (4) comparing the amount of circulating EBV DNA present in the blood sample to a control.

Preferably, the blood sample is a non-cellular fluid sample. By non-cellular we mean that the sample is either blood sera where the cells are extracted by clotting and separation of the cells from the remaining fluid or by inhibiting clotting and centrifuging the fluid fraction (plasma). The EBV DNA is measured from the fluid fraction. When EBV is found in the fluid of a non-cellular sample, it is understood that the infection is active and infected cells releasing EBV.

In a second aspect, the present invention features a genotyping test for detecting known polymorphisms that divides EBV into malignant and benign subtypes.

In a third aspect, the present invention features diagnostic kits comprising suitable reagents for detecting EBV DNA and EBV subtypes in the serum or plasma of patients. The kits according to the present invention may further comprise one or more of a device for obtaining a blood sample from a patient, a means to separate the EBV DNA from the blood sample and a means to quantify the amount of EBV DNA present in the blood sample. Such kits are useful for diagnosing, detecting, monitoring and determining the prognosis for EBV associated cancers including lymphomas.

## DETAILED DESCRIPTION OF THE INVENTION

The present invention features methods for predicting, diagnosing, detecting, monitoring and determining the prognosis of EBV associated cancers in a patient. The methods feature detecting or determining the amount of EBV DNA present in the serum of these patients and if positive, a second method determines the cancerous potential of the EBV subtype in these patients. The methods according to the present invention have broad applicability in clinical medicine since latent infection of EBV is prevalent and widespread. In some population, over 90% of the population has such a latent EBV infection.

Clinically, circulating EBV DNA is applicable in diagnosing and monitoring gastric carcinoma patients who have EBER-positive tumors, similar to what has been achieved for nasopharyngeal cancers (Lo, Y.M.D. *et al.*, Clin. Cancer Res., 59: 1188-1191 (1999); Lo, Y.M.D. *et al.*, Cancer Res., 59: 5452-5455 (1999)) and certain lymphomas (Lei, K.I. *et al.*, Br. J. Haematol., 111: 239-246 (2000); Drouet, E. *et al.*, J. Med. Virol., 57: 383-389 (1999); Gallagher, A. *et al.*, Int. J. Cancer, 84: 442-448 (1999)). The recent demonstration of the prognostic significance of circulating EBV DNA in nasopharyngeal cancers (Lo, Y.M.D. *et al.*, Cancer Res., 60: 6878-6881) suggests that EBV DNA measurement has prognostic importance for gastric carcinoma.

The methods according to the present invention are also applicable for detecting, monitoring and determining the prognosis of EBV associated cancers apart from head, neck and lymphoid malignancies where those cancers are associated with EBV. Some of these neoplasms have been shown previously to be associated with EBV infection (Bonnet *et al.*, J Natl Cancer Inst. 91: 1376-81 (1999)), as have certain liver cancers (Sugawara *et al.*, Virology. 256: 196-202 (1999)).

Any of the conventional DNA amplification or signal amplification methods may be used for detection of EBV DNA. In most instances, it is desirable to amplify the target sequence using any of several nucleic acid amplification procedures which are well known in the art. Specifically, nucleic acid amplification is the enzymatic synthesis of nucleic acid amplicons (copies) which contain a sequence that is complementary to a nucleic acid sequence being amplified. Examples of nucleic acid amplification procedures practiced in the art include the polymerase chain reaction (PCR), strand displacement amplification (SDA), ligase chain reaction (LCR), and transcription-associated amplification (TAA). Nucleic acid amplification is especially beneficial when the amount of target sequence present in a sample is very low. By amplifying the target sequences and detecting the amplicon synthesized, the sensitivity of an assay can be vastly improved, since fewer target sequences are needed at the beginning of the assay to better ensure detection of nucleic acid in the sample belonging to the organism or virus of interest.

Methods of nucleic acid amplification are thoroughly described in the literature. PCR amplification, for instance, is described by Mullis et al. in U.S. Pat. Nos. 4,683,195 Methods of nucleic acid amplification are thoroughly described in the literature. PCR amplification, for instance, is described by Mullis et al. in U.S. Pat. Nos. 4,683,195, 4,683,202 and 4,800,159, and in *Methods in Enzymology*, 155: 335-350 (1987). Examples of SDA can be found in Walker, *PCR Methods and Applications,* 3: 25-30 (1993), Walker et al. in *Nucleic Acids Res.,* 20: 1691-1996 (1992) and *Proc. Natl. Acad. Sci.*, 89: 392-396 (1991). LCR is described in U.S. Pat. Nos. 5,427,930 and 5,686,272. And different TAA formats are provided in publications such as Burg et al. in U.S. Pat. Nos. 5,437,990; Kacian et al. in U. S. Pat. Nos. 5,399,491 and 5,554,516; and Gingeras et al. in International Application No. PCT/US87/01966 and International Publication No. WO 88/01302, and International Application No. PCT/US88/02108 and International Publication No. WO 88/10315.

Real-time quantitative PCR is a preferred means to monitor EBV DNA and is based on the continuous optical monitoring of the progress of a fluorogenic PCR reaction (Heide *et al. Genome Res.* 6: 986-694, 1996 and Lo *et al. Am J. Hum. Genet.* 62: 768-775, 1998). In this system, in addition to the two amplification primers used in conventional PCR, a dual-labeled fluorogenic hybridization probe is also included (Livak, *et al. PCR Methods Appl.,* 4357-362, 1995). One fluorescent dye serves as a reporter (FAM), and its emission spectrum is quenched by a second fluorescent dye (TAMRA). During the extension phase of PCR, the 5' to 3' exonuclease activity of the *Taq* DNA ploymerase (9) cleaves the reporter from the probe, thus releasing it from the quencher and resulting in an increase in fluorescence emission at 518 nm.

The methods according to the present invention generally comprise the steps of (1) obtaining a blood sample from a patient, (2) extracting DNA from the blood sample, (3) measuring the amount of circulating EBV DNA present in the blood sample, and (4) comparing the amount of circulating EBV DNA present in the blood sample to a control. Preferably, the blood sample is centrifuged, a fluid fraction is obtained, and the EBV DNA is measured from the fluid fraction.

Those of skill in the art will understand that the DNA may be extracted from a blood sample by many means known in the art. One preferred means is using a QIAamp Blood Kit. Also, the amount of circulating EBV DNA may be measured using one of many known or novel protocols. A protocol comprising a real time PCR amplification system is particularly preferred. Standard procedures for comparing the levels of EBV DNA so detected to a control may easily be devised so as to statistically assess the significance of the values obtained.

The number of copies of EBV DNA may be measured over time and correlated to disease progression or regression. Thereby, the present invention provides a non-invasive method that allows a good measurement of the prognosis of these EBV associated cancers.

On the other hand, because of the frequent presence of lytic reactivations of EBV as well as transformation of chronic active EBV carriers, EBV DNA level can be found in a significant percentage of the population without cancer. In our study quoted in the patent claiming priority, 3.6% of the normal population is positive in serum or plasma EBV DNA at any one time. Fortunately, most of these cases, especially those with EBV DNA copies of less than 500 copies/ml, are in fact benign in origin. The level of EBV DNA will go down to zero in these benign cases after one to two weeks, marking the end of the lytic reactivation or chronic reinfection. Nevertheless such reactivation of EBV and resulting elevation of IgA antibodies against EBV capsid antigen and neutralizing antibodies against EBV Dnase are predictive of nasopharyngeal carcinoma in a population in Taiwan. ( Chien YC, *Serologic markers of EBV infection and nasopharyngeal carcinoma in Taiwanese men, N England J Med, Vol 345 no26 Dec 27, 2001)* Conclusively, it is common to find such low-grade EBV DNA infections and patients are obviously anxious to know their carcinogenic potential. In fact, our serum or plasma EBV DNA test, while extremely sensitive and useful to detect and monitor cancer such as nasopharyngeal carcinoma, has been partially responsible for finding such a large portion of the population that in fact is harboring and reactivating EBV at very frequent intervals. The present invention is to turn this extreme sensitivity into a useful tool so that the carcinogenic potential of these frequent EBV reactivators can be measured once and for all.

In a second aspect, the present invention features genotyping methods that can detect the different subtypes of EBV with different carcinogenic potentials. One of the methods and genotyping that was published showed that polymorphisms in the regulatory sequences of BZLF1 promoter region of the lytic regulatory gene of EBV are differentially distributed among malignant and nonmalignant cells. Three polymorphic Zp sequences were detected. Among the malignant samples, all 52 samples showed polymorphisms either as Zp-P (identical to the EBV B95.8 strain) or the Zp-V3 sequence. For the benign samples, all 52 showed a Zp-V4 polymorphism. (Gutierrez, *Discrete alterations in the BZLF1 promoter in tumor and non tumor associated EBV, J of the National Cancer Institute, Vol 94, no 23 December 4 2002*)

In the third aspect, diagnostic kits comprising suitable reagents for detecting EBV DNA in the serum or plasma of patients and for the polymorphic subtypes are included. The kits according to the present invention may further comprise one or more of a device for obtaining a blood sample from a patient, a means to separate the EBV DNA from the blood sample and a means to quantify the amount of EBV DNA present in the blood sample. Such kits are useful for diagnosing, detecting, monitoring and determining the prognosis of EBV associated cancers.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

EXAPMPLES

The following examples are provided by way of illustration only and not by way of limitation. Those of skill will readily recognize a variety of noncritical parameters which could be changed or modified to yield essentially similar results.

**EXAMPLE 1**

**Materials and Methods**

**Detecting EBV DNA from Plasma Samples** Patients are recruited with known EBV associated cancers such as nasopharyngeal carcinoma. Normal individuals are also recruited that serve as control for benign carriers of EBV and for the detection of serum or plasma EBV DNA and for EBV subtyping according to polymorphisms.

DNA Extraction from Plasma Samples is done as followed. Peripheral blood (5 ml) can be collected from each subject into an EDTA tube for the isolation of plasma. Blood samples are centrifuged at 1600 X g, and plasma carefully removed from the EDTA-containing tubes and transferred into plain polypropylene tubes. The samples are stored at -20°C until further processing. DNA form plasma samples are extracted using a QIAamp Blood Kit (Qiagen, Hilden, Germany) using the blood and body fluid protocol as recommended by the manufacturer (2). Plasma samples (130-800 µl/column) are used for DNA extraction. The exact amount is documented for the calculation of the target DNA concentration. A final elution volumn of 50 µl is used from the extraction columns.

Circulating EBV DNA concentrations were measured using a real time quantitative PCR system towards the BamHI-W fragment region of the EBV genome (Lo, Y.M.D. *et al*., *Cancer Res.,* 59: 1188-1191 (1999)). The principals of real time quantitative PCR and reaction set-up procedures were as previously described (Lo, Y.M.D. *et al*., *Cancer Res.,* 59: 1188-1191 (1999)). Data were collected using an ABI Prism 7700 Sequence Detector and were analyzed using the Sequence Detection System software (version 1.6.3) developed by Applied Biosystems. Results were expressed as copies of EBV genomes per milliliter of serum.

All serum DNA samples were also subjected to real time PCR analysis for the (beta-globin gene (Lo, Y.M.D. *et al*., *Cancer Res*., 59: 1188-1191 (1999)), which gave a positive signal on all tested samples, thus demonstrating the quality of the extracted DNA. Multiple negative water blanks were included in every analysis.

More specifically, two real-time quantitative PCR systems have been developed for EBV DNA detection: (a) one toward the *Bam*HI-W region; and (b) the other toward the *EBNA-I* region (Baer, et al Nature, 310: 207-211, 1984). The *Bam*HI-W system consisted of the amplification primers (SEQ ID NO: 1) W-44F (5'-CCCAACACTCCACCACACC-3') and (SEQ ID NO: 2) W-119R (5'-TCTT AGGAGCTGTCCGAGGG-3') and the dual-labeled fluorescent probe (SEQ ID NO: 3) W-67T (5'-FAM)CACACACTACACACACCCAC-CCGTCTC(TAMRA)-3']. The *EBNA-1* system consisted of the amplification primers (SEQ ID NO: 4) EBNA-1162F (5'-TCATCATCATCCGGGTCTCC-3') and (SEQ ID NO: 5) EBNA-1229R (5'-CCTACAGGGT-GGAAAAATGGC-3') and the dual-labeled fluorescent probe (SEQ ID NO: 6) EBNA-1186T [5'-(FAM)CGCAGGCCCCCTCCAGGTA-GAA(TAMRA)-3']. The fluorescent probes contained a 3'-blocking phosphate group to prevent probe extension during PCR. Primer/probe combinations were designed using Primer Express software (Perkin-Elmer Corp., Foster City, CA). Sequence data for the EBV genome were obtained from the GenBank Sequence Database (accession number V01555). Real-time quantitative PCR for the β-*globin* gene consisted of primers and probe, as described previously in Lo, *et al*. *Am J. Hum Genet* 62: 768-775, 1998), and was used as a control for the amplifiability of plasma DNA.

Fluorogenic PCR reactions are set up in a reaction volume of 50 µl using components (except for the fluorescent probes and amplification primers) supplied in a TaqMan PCR Core Reagent Kit (Perkin-Elmer Corp.). Fluorescent probes are custom-synthesized by Perkin-Elmer Applied Biosystems. PCR primers were synthesized by Life Technologies, Inc. (Gaithersburg, MD). Each reaction contained 5 µl of 10x buffer A; 300 nM of each of the amplification primers; 25 nM (for the EBV probes) or 100 nM (for the β-*globin* probe) of the corresponding fluorescent probe; 4 MM MgCl$_2$; 200 µm each of dATP, dCTP, and dGTP; 400 µM dUTP; 1.25 units of AmpliTaq Gold; and 0.5 unit of AmpErase uracil N-glycosylase.

DNA amplifications are carried out in a 96-well reaction plate format in a Perkin-Elmer Applied Biosystems 7700 Sequence Detector. Each sample are analyzed in duplicate. Multiple negative water blanks were included in every analysis.

A calibration curve is run in parallel and in duplicate with each analysis, using DNA extracted from the EBV-positive cell line Namalwa (American Type Culture Collection CRL-1432; See Klein *et al*., *Int J. Cancer,* 10: 44-57, 1972) as a standard. Namalwa is a diploid cell line that contains two integrated viral genomes/cell. A conversion factor of 6.6 pg of DNA/diploid cell was used for copy number calculation (Saiki et al., Science, 239: 487-491, 1988). Concentrations of circulating cell-free EBV DNA were expressed as copies of EBV genome/ml plasma.

An identical thermal profile was used for the EBV *Bam*HI-W and *EBNA-I* PCR systems. Thermal cycling was initiated with a 2-min incubation at 50°C for the uracil N-glycosylase to act, followed by an initial denaturation step of 10 min at 95°C, and then 40 cycles of 95°C for 15 s and 56°C for 1 min were carried out.

Amplification data collected by the 7700 Sequence Detector and stored in a Macintosh computer (Apple Computer, Cupertino, CA) is then analyzed using the Sequence Detection System software developed by Perkin-Elmer Applied Biosystems. The mean quantity of each duplicate is used for further concentration calculation. The plasma concentration of EBV DNA or the β-*globin* gene (expressed in copies/ml) is calculated using the following equation:

$$C = Q \times \frac{V_{DNA}}{V_{PCR}} \times \frac{1}{V_{ext}}$$

in which *C* represents the target concentration in plasma (copies/ml), *Q* represents the target quantity (copies) determined by a sequence detector in a PCR, $V_{DNA}$ represents the total volume of DNA obtained after extraction (typically 50 µl/Qiagen extraction), $V_{PCR}$ represents the volume of DNA solution used for PCR (typically 5 µl, and $V_{ext}$ represents the volume of plasma/serum extracted (typically 0.13-0.80 ml)).

The presence of EBV in tumor cells was assessed by in-situ hybridization on paraffin-embedded tissue sections using a fluorescein-conjugated oligonucleotide probe for EBER (Novocastra, U.K.) as previously described (Hui, P.K. *et al.*, *Hum. Pathol.,* 25: 947-952 (1994)).

Detection of EBV Subtypes Patients' samples DNA can be taken from the cell free plasma as described in [27]. These DNA samples can also be isolated, by using a standard phenol/chloroform extraction technique after proteinase K digestion, from peripheral blood lymphocytes. Furthermore, DNA samples can also be isolated from suspicious areas of EBV tumors or reactivation sites.

We use the TaqMan Allelic Discrimination assay with the 5' nuclease activity of Taq polymerase to detect a fluorescent reporter signal generated during or after PCR reactions. For genotyping of EBV, one pair of TaqMan probes and one pair of PCR primers are used. The assay uses two TaqMan probes that differ at the polymorphic site, with one probe complementary to the wide-type allele and the other to the variant allele. A 5' reporter dye and a 3' quencher dye are covalently linked to the wild-type or variant allele probes. When the probes are intact, fluorescence is quenched because of the physical proximity of the reporter and quencher dyes. During the PCR annealing step, the TaqMan probes hybridize to the targeted polymorphic site. During the PCR extension phase, the 5' reporter dye is cleaved by the 5' nuclease activity of the Taq polymerase, leading to an increase in the characteristic fluorescence of the reporter dye. Specific genotyping is determined by measuring the signal intensity of the two different reporter dyes after the PCR reaction. TaqMan genotyping instrumentation and reagents are supported by Applied Biosystems.

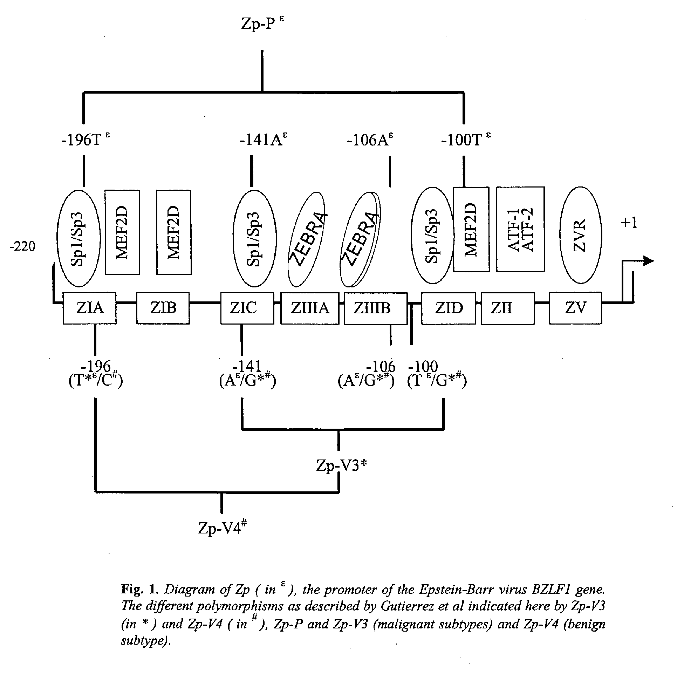

EBV subtypes polymorphisms tested on the 200-nt Zp BZLF1 promoter region are listed as follows (as shown in Fig. 1):

(1). Zp-P (protype) identical to EBV strain B95-8
(2) Zp-V3 with three point changes:

(a) A to G at position -141
(b) A to G at position -106
(c) T to G at position -100

(3) Zp-V4: containing all of the Zp-V3 plus a fourth substitution, T to C at position -196

## Clinical Results Using the Comparison of V3 (Malignant) and P (Benign) Status of Patient Samples in Nasophayrngeal Carcinoma (NPC)

### BZLF1 real-time PCR Reaction Mix

**Vp-P = FAM**
**Vp-V3 = VIC**

| Reagent | Volume (30 ul) |
|---|---|
| 10x buffer A | 3.00 |
| 25 mM Mg2+ | 4.50 |
| Dntp | 2.50 |
| BZLF1 (Forward primer) | 1.00 |
| BZLF1 (Reverse primer) | 1.00 |
| BZLF1-probe P | 0.40 |
| BZLF1-probe V3 | 0.40 |
| UNG | 0.30 |
| Taq gold | 0.15 |
| H2O | 11.85 |
| Total: | 25.00 |

**25 ul Master Mix + 5 ul DNA sample**

**BZLF1 Real-time PCR Profile**

| 50°C | 2 mins |
|------|--------|
| 95°C | 5 mins |
| 94°C | 20 secs |
| 60°C | 1 min |

X 45 cycles

| Patient No. | EBV DNA (copies/ml plasma) | NPC | ? NPC | EBonco (V3 or P) | Additional Clinical Information |
|-------------|----------------------------|-----|-------|------------------|-------------------------------|
| 375 | 101 - 1000 | | X | P | |
| 405 | 195 | X | | P | |
| 709 | 34 | X | | P | NPC (new case) |
| 837 | 1174 | X | | P | |
| 931 | 1279 | X | | P | |
| 1024 | 605 | X | | P | |
| 256 | 2273312 | X | | V3 | Metastatic NPC |
| 262 | 127971 | X | | V3 | |
| 331 | <100 | X | | V3 | Recurrence of NPC |
| 348 | 22250 | X | | V3 | |
| 350 | 1050 | X | | V3 | |
| 377 | 33925 | | X | V3 | |
| 399 | 101 - 1000 | | X | V3 | Cervical LN also positive |
| 404 | 304 | X | | V3 | |
| 408 | 295 | | X | V3 | |
| 427 | 106 | X | | V3 | NPC post RT with known bone & lung Secondary |
| 447 | 3000 | X | | V3 | |
| 735 | 59 | X | | V3 | Metastatic NPC |
| 785 | 13631 | X | | V3 | Metastatic NPC |
| 790 | 43 | X | | V3 | |
| 858 | 778 | X | | V3 | |
| 960 | 944 | X | | V3 | |
| 991 | 4650 | X | | V3 | |
| 1003 | 101 - 1000 | X | | V3 | |
| 1025 | 5131 | X | | V3 | |
| TH589 | 25 | X | | V3 | |

| Patient No. | EBV DNA (copies/ml plasma) | Normal | EBonco Results (V3 or P) |
|---|---|---|---|
| 622 | 0 | X | Undetermined |
| 623 | 0 | X | Undetermined |
| 624 | 0 | X | Undetermined |
| 625 | 0 | X | Undetermined |
| 626 | 0 | X | Undetermined |
| 627 | 0 | X | Undetermined |
| 628 | 0 | X | Undetermined |
| 629 | 0 | X | Undetermined |
| 634 | 0 | X | Undetermined |
| 635 | 0 | X | Undetermined |
| 980 | 0 | X | Undetermined |
| 981 | 0 | X | Undetermined |
| 982 | 0 | X | Undetermined |
| 1033 | 0 | X | Undetermined |
| 1034 | 0 | X | Undetermined |
| 1035 | 0 | X | Undetermined |
| TH39 | 0 | X | Undetermined |
| TH355 | 0 | X | Undetermined |
| TH516 | 0 | X | Undetermined |
| TH663 | 0 | X | Undetermined |
| TH836 | 0 | X | Undetermined |
| TH1142 | 0 | X | Undetermined |
| TH 1164 | 0 | X | Undetermined |

**Conclusion**

It can be shown from the above clinical study that out of 26 patients with NPC and possible NPC (no pathological confirmation), 20 carried the more malignant subtype of V3 (for Chinese population, V3 is the dominant malignant subtype), contributing to 77% of the NPC population. If the possible NPC cases were deleted from the study, the percentage of the V3 subtype remained the same as 77% (17 out of 22 patients). For normal individuals with no EBV DNA circulating in the blood, there is no V3 or P detected. Preliminary study shown here demonstrated that the combination or individual blood testing of the subtype V3 of the EBV DNA may be a powerful tool for the future estimation of cancer changes in patients with a positive EBV DNA titer in their blood.

Alternatively detection of EBV subtypes can be done by other methods which enable single base discrimination, including, but not limited to single-strand conformation polymorphism, allele-specific PCR, mass spectrometric analysis of

PCR products, artificial restriction fragment length polymorphism, ligase chain reaction. The following protocol illustrates the use of single-strand conformation polymorphism analysis. Five hundred nanograms of genomic DNA obtained from all tumor and nontumor samples was directly used as a template in polymerase chain reactions (PCRs). PCR was used to amplify the fragment from nucleotides -221 to +12 (with respect to the transcription start site) of the BZLF1 promoter (nucleotides 103420-103182 of the EBV gonome, Genbank accession no. NC_001345). The primers used were 5'-agcatgccatgcatatttc-3' and 5'-ttggcaaggtgcaatgttt-3'. PCR conditions consisted of 5 minutes at 95°C, 30 cycles of 30 seconds at 95°C, 30 seconds at 60°C, and 1 minute at 72°C, followed by a long extension of 10 minutes at 72°C. [$^{32}$P]dCTP was included in the PCR buffer. PCR products were separated by electrophoresis through 6% nondenaturing acrylamide gels (19:1, acrylamide to bis) containing 10% glycerol at 6W for 18 hours and visualized by autoradiography. Autoradiograms were exposed for 2-16 hours. Single-strand conformation polymorphisms (SSCPs) were apparent by differences in the patterns of migration of the PCR product. Amplified product obtained from the EBV strain B95.8 served as a reference control. The above protocol demonstrates that other cancer causing subtypes can be found and be tested similarly from blood samples as shown in the clinical study illustrated for V3 and P.

**Conclusions**

EBV samples of NPC patients in the Chinese population show that the Zp-V3 subtype may diagnosis patients that are more cancer prone than those with Zp-P subtype.

**Results**

Results from our past publications showed a sensitivity of 96% and a specificity of 93% in detecting nasopharyngeal carcinoma with concentration of EBV DNA copies exceeding 500 copies/ml. Among the healthy control, 3.6% showed a positive EBV DNA titre, most of them below 500 copies/ml. This present invention can predict further that among the 3.6% of the health population, how many are actually harboring a subtype of EBV that can most likely link to the development of EBV associated cancer. More careful assessment of the patient, and detail follow up may be able to pick up EBV associated cancer at early stage, whereby a cure is expected.

**Discussion**

Clinically, circulating EBV DNA may have application in the diagnosis and monitoring in the proportion of gastric carcinoma patients who have EBER-positive tumors, similar to what has been achieved for NPC (Lo, Y.M.D. *et al*., *Cancer Res*., 59: 1188-1191 (1999); Lo, Y.M.D. *et al*., *Cancer Res., 59:* 5452-5455 (1999)) and certain lymphomas (Lei, K.I. *et al*., *Br. J. Haematol.,* 111: 239-246 (2000); Drouet, E, *et al*., *J. Med*. *Virol*. 57: 383-389 (1999); Gallagher, A. *et al*., *Int*. *J. Cancer,* 84: 442-448 (1999)). Recently, the value of circulating EBV DNA in nasopharyngeal cancer prognosis has been demonstrated. Additional data (Lo, Y.M.D. *et al*., *Cancer Res.,* 60: 6878-6881) indicate that EBV DNA measurement also has prognostic importance for gastric carcinoma.

There is however, a large group of patients that have been diagnosed with low EBV DNA level and chronic or frequent EBV reactivations with no visible sign and symptom of cancers. Disposition and follow up of such patients is so far unsatisfactory due to the known carcinogenic potential of EBV with additional information that cancer is indeed more prevalent in this group.

One of the many methods to detect polymorphism or other genotypic abnormality is demonstrated by Gutierrez ( *JNCI, 94 no23 Dec 4th 2002* ). Her publication revealed the division of the promoter region of the EBV lytic gene BZLF1 into three subtypes with polymorphism that can be detected by PCR-single strand confirmation polymorphism. All cancer samples belonged to two subtypes of Zp-P and Zp-V3. All benign samples belonged to one subtype Zp-V4. In the Chinese population, however, the Zp-V3 is the dominant malignant subtype over Zp-P, while Zp-V4 cannot be found.

The very sensitive method described in this invention can detect cell free plasma EBV DNA in a group of individuals that may only have apparent benign diseases such as lytic reactivation in the upper airways and gastritis. They usually have lower than 500 copies/ml of EBV DNA and exhaustive examination reveals no known malignancy. The combination of the second part of this invention through the detection of definitive polymorphisms and subtypes of EBV will further clarify the management of this group of individuals. One group with the malignant subtype should undergo regular follow up and frequent measurement of the EBV DNA level. The other group with the benign subtype may be relieved of their anxiety and be followed up on a as needed basis.

Data also suggest that circulating EBV DNA may be useful in many other cancer types that are associated with EBV. Examples of such cancers include breast cancer (Bonnet, M. *et al*., *J. Natl. Cancer Inst.,* 91: 1376-1381 (1999)) and hepatocellular carcinoma (Sugawara, Y. *et al*., *Virology*, 256: 196-202 (1999)). As the association between some tumor types and EBV is still controversial, the possible detection of EBV DNA and the detection of cancerous subtypes in the plasma of patients with such tumors may contribute towards resolving these issues.

**Claims**

1. A method of determining the probability of a patient with Epstein Barr virus DNA in blood for the prognosis and diagnosis of Epstein Barr virus associated cancers by either a) and b) in combination or b) alone whereby a) and b) are defined as below:

   (a) first screening such a patient with EBV DNA described herein using serum or plasma samples and then
   (b) confirming such a probability by determining the EBV subtypes according to polymorphisms in the blood plasma or serum.

2. A method of claim 1 wherein the patient can be diagnosed with Epstein Barr virus associated cancers and the cancer cells are free of EBV nucleic acid.

3. A method of claim 1 wherein the patient can be diagnosed with Epstein Barr virus associated cancers and the cancer cells contain EBV nucleic acid.

4. A method of claim 1 wherein the EBV DNA screening is done through blood plasma or serum while the EBV subtyping of polymorphisms is done through the white cells including the lymphocytes.

5. A method of claim 1 wherein the EBV DNA screening is done through blood plasma or serum while the EBV subtyping of polymorphisms is done through other body tissue or tissues suspicious of harboring the virus.

6. A method of claim 1 wherein the EBV subtyping of polymorphisms is directed through other variations at the DNA level apart from single nucleotide changes, such as but not exclusively consisted of insertion or deletions (indels) and variations in the number of tandem repeats (VNTR).

7. A method of claim 6 wherein the single nucleotide changes are different than the ones mentioned in the materials and methods.

8. A method of claim 1, wherein the method of detecting EBV subtyping of polymorphisms is not limited to the TaqMan Allelic Discrimination Assay, Single Strand Confirmation Polymorphism, but can be of any method such as but not exclusively consisted of PCR-Restriction Fragment Length Polymorphism Analysis, Oligonucleotide Ligation Assay Genotyping, Minisequencing, Fluorescence Resonance Energy Transfer Detection, Invader Assay and Allele-Specific Ligation, etc.

9. A method of diagnosis, detecting, monitoring and determining the prognosis of Epstein Barr virus associated cancer in a patient comprising the step of detecting EBV DNA and then EBV subtypes present in the serum of plasma of the patient.

10. The method of claim 9 comprising the steps of:

    (1) obtaining a blood sample from a patient;
    (2) obtaining a fluid fraction from the blood sample;
    (3) extracting DNA from the fluid fraction; and
    (4) measuring the amount of circulating EBV DNA present in the fluid fraction.

11. The method of claim 10 further comprising the step of:

(5) comparing the amount of circulating EBV DNA present in the fluid fraction to a control.
(6) comparing the EBV subtypes after its determination to ones that are defined as either mostly malignant or benign.

12. A kit for determining the increased probability of a patient with increase EBV DNA in blood for the prognosis and diagnosis of Epstein Barr virus associated cancers.

(a) nucleic acid for detecting Epstein Barr virus in the blood of patients suffering from; and
(b) instructions for use of the nucleic acid to determine the presence or absence of Epstein Barr virus and an explanation of the increased probability of the patient suffering from Epstein Barr virus associated cancers if confirmed with EBV subtyping.
(c) TaqMan probes and PCR primers with two TaqMan probes differ at the polymorphic site with one complementary to the benign and the other to the malignant allele. The number of different malignant and benign subtypes will dictate the number of pairs of TaqMan probes.

13. A diagnostic kit according to claim 12 comprising a device for obtaining a blood sample from a patient.

14. A diagnostic kit according to claim 12 comprising a means to separate EBV DNA from a blood sample or from other specimen.

**Fig. 1.** *Diagram of Zp ( in $^{\varepsilon}$ ), the promoter of the Epstein-Barr virus BZLF1 gene. The different polymorphisms as described by Gutierrez et al indicated here by Zp-V3 (in * ) and Zp-V4 ( in $^{\#}$ ), Zp-P and Zp-V3 (malignant subtypes) and Zp-V4 (benign subtype).*